# EUROPEAN PATENT SPECIFICATION

(11) **EP 2 611 402 B1**
(45) Date of publication and mention of the grant of the patent: **09.08.2017**
(21) Application number: 11821906.2
(22) Date of filing: 25.08.2011
(51) Int. Cl.: A61F 13/00, A61F 13/15, A61F 13/53, A61F 13/534, A61F 13/475, A61F 13/533

(54) **INCONTINENCE LINER**
INKONTINENZAUSKLEIDUNG
COUCHE-CULOTTE D'INCONTINENCE

(30) Priority: 31.08.2010 JP 2010194643
(43) Date of publication of application: 10.07.2013
(73) Proprietor: Unicharm Corporation, Ehime 799-0111 (JP)
(72) Inventor: KAWAKAMI, Yusuke, Kanonji-shi Kagawa 769-1602 (JP)
(74) Representative: Dolleymores
(86) International application number: PCT/JP2011/069860
(87) International publication number: WO 2012/029891

(56) References cited:
- EP-A1- 1 917 939
- EP-A1- 2 092 918
- EP-A1- 2 130 521
- EP-A1- 2 130 522
- WO-A1-00/32145
- JP-A- 2006 230 714
- JP-A- 2008 178 667
- JP-A- 2009 000 173
- JP-A- 2009 056 269
- JP-A- 2009 100 846

## Description

### Technical Field

The present invention (disclosure) relates to an incontinence liner.

### Background Art

Known in the art is an absorptive product provided with a liquid permeable skin contact side sheet, a liquid impermeable non-skin contact side sheet, and an absorptive body which is arranged between these skin contact side sheet and non-skin contact side sheet and which includes an absorptive material (see PLT 1). In the absorptive product, the absorptive body is provided with a center thick part and a thin part surrounding the thick part. The liquid to be absorbed is absorbed from the thick part in the absorptive body, then diffuses from the thick part in the thin part. By doing this, the liquid to be absorbed can be quickly taken into the absorptive body and therefore the possibility of the liquid to be absorbed leaking without being absorbed in the absorptive body can be reduced.

### Citation List

### Patent Literature

PLT 1: Japanese Patent Publication (A) No. 2008-237382
PLT 2: WO 00/32145 A1
PLT 3: EP 1917939 A1

### Summary of Invention

### Technical Problem

In the above PLT 1, if considering the quick absorption of the liquid to be absorbed at the thick part, it is considered preferable that the liquid to be absorbed quickly diffuse in the thin part.

However, if the liquid to be absorbed excessively quickly diffuses in the thin part, the liquid to be absorbed might leak from the peripheral edge of the absorptive body. That is, it might be necessary to restrain the speed of diffusion of the liquid to be absorbed in the absorptive body. Urine has a viscosity lower than that of menstrual blood, so when the absorptive product is an incontinence liner and the liquid to be absorbed is mainly urine, the likelihood of leakage is higher than when the absorptive product is a sanitary napkin and the liquid to be absorbed is mainly menstrual blood.

Both PTL 2 and PTL 3 disclose sanitary napkins having a channel or emboss formed in the absorptive body.

### Solution to Problem

The present invention provides the incontinence liner of independent Claim 1. The dependent claims specify preferred but optional features.

### Advantageous Effects of Invention

It is possible to secure quick diffusion of the liquid to be absorbed in the absorptive body while suppressing leakage of the liquid to be absorbed.

### Brief Description of the Drawings

FIG. 1 is a plan view of an incontinence liner in accordance with the present invention.
FIG. 2 is a longitudinal cross-sectional view taken along line M-M of FIG. 1.
FIG. 3 is a back view of the incontinence liner in accordance with the present invention.
FIG. 4 is a plan view of an absorptive body of the incontinence liner in accordance with the present invention.
FIG. 5 is a schematic view explaining the flow of the liquid to be absorbed in the absorptive body.
FIG. 6 is a partial schematic longitudinal cross-sectional view of diffusion-suppressing parts and a diffusing part in the absorptive body.
FIG. 7 is a partial schematic longitudinal cross-sectional view of a compression groove in the absorptive body.

### Description of Embodiments

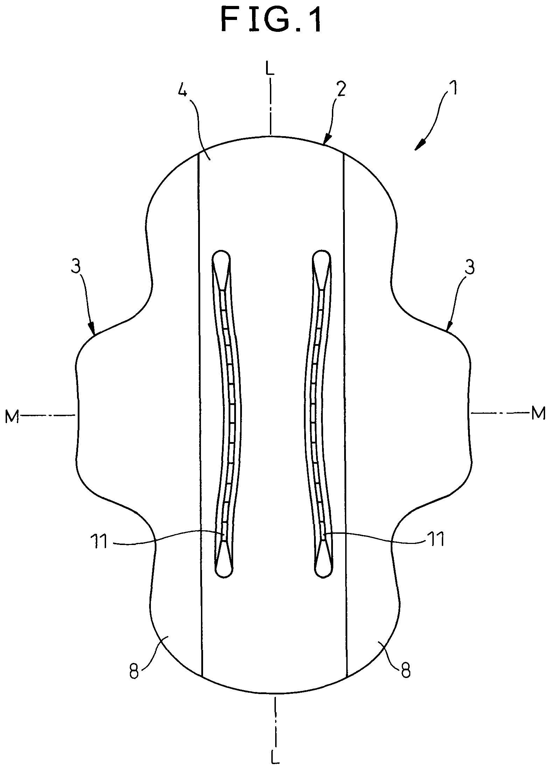

Referring to FIG. 1, the incontinence liner 1 is provided with an oval-shaped main body 2 and a pair of wings 3 which project outwardly from the side parts of the main body 2 in the width direction. The present invention is not to be limited to this shape of main body or to the presence of wings.

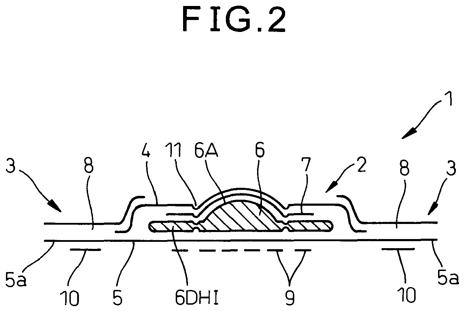

In particular, as shown in FIG. 2, the main body 2 is comprised of a skin contact side sheet (skin contacting sheet) 4 and a non-skin contact side sheet (non-skin contacting sheet) 5 superposed on each other, an absorptive body 6 arranged between these skin contact side sheet 4 and non-skin contact side sheet 5, and a cushion sheet 7 arranged between the skin contact side sheet 4 and the absorptive body 6. On the other hand, the wings 3 are comprised of parts 5a of the non-skin contact side sheet 5 extending outwardly beyond the side edges of the skin contact side sheet 4 in the width direction and side sheets 8 superposed on the parts 5a. Here, the skin contact side sheet 4 and non-skin contact side sheet 5, and the non-skin contact side sheet parts 5a and side sheets 8 are respectively joined by, for example, a hot melt adhesive, heat sealing, etc. Note that, in an embodiment of the present invention, the side sheets 8 are also superposed and joined with the skin contact side sheet 4, and therefore, the main body 2 also includes the side sheets 8. In further embodiments, the non-skin contact side sheet 5 may also not extend up to the wings 3. In this case, the wings 3 may be comprised of only the side sheets 8, or the side sheets 8 and other sheets superposed with the same.

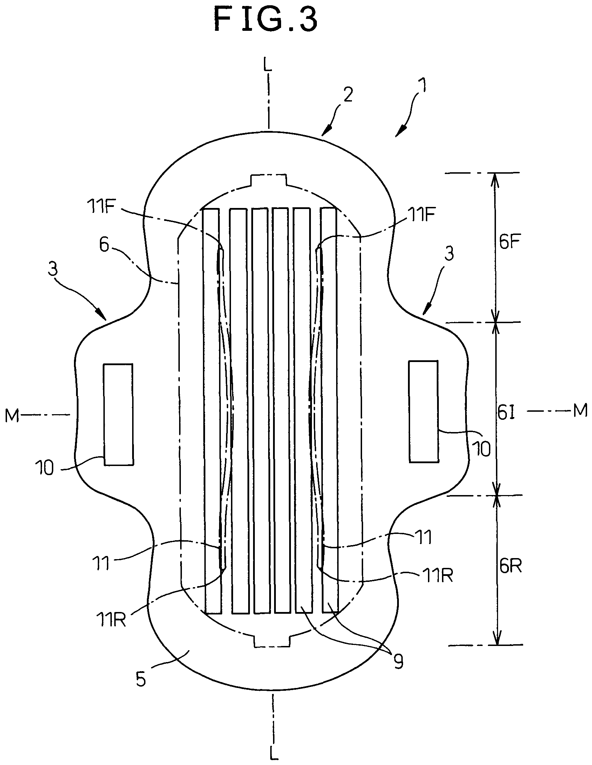

Further, as shown in FIG. 2 and FIG. 3, at the outer surface of the non-skin contact side sheet 5 corresponding to the back surface of the main body 2, adhesives 9, e.g., adhesive stripes, separated from each other in the width direction and extending in parallel in the longitudinal direction are applied, while at the outer surface of the non-skin contact side sheet 5 corresponding to the back surfaces of the wings 3, adhesives 10 are applied. The present invention is not to be limited to the use of adhesives, nor to the arrangement of adhesives as described herein.

Further, the liner 1 is formed with a pair of compression grooves 11 extending from the skin contact side sheet 4 to the absorptive body 6. In an embodiment according to the present invention, each of the compression grooves 11 is comprised of deep groove sections and shallow groove sections, which alternately repeat in the length (longitudinal) direction. In some embodiments, the compression grooves 11 may also be comprised of deep grooves which continue in the length direction.

Note that, in an embodiment according to the present invention, the liner 1 is formed symmetrically about a longitudinal direction center line L-L and a width direction center line M-M. In some embodiments, the liner 1 is formed symmetrically only about the longitudinal direction center line L-L.

The skin contact side sheet 4 has liquid permeability and is, for example, comprised of a woven fabric or nonwoven fabric made of polyethylene (PE), polypropylene (PP), polyethylene terephthalate, or other polyolefin-based thermoplastic hydrophobic fiber treated to make it hydrophilic, or is comprised of pulp, cotton, or other natural fiber or rayon or another cellulose fiber. In an embodiment according to the present invention, as the skin contact side sheet 4, a 20 to 40 g/m² air-through nonwoven fabric comprised of PE/PP fiber is used.

The non-skin contact side sheet 5 has liquid impermeability and moisture permeability and is, for example, comprised of a hydrophobic nonwoven fabric, a water impermeable plastic film, a laminate sheet of a nonwoven fabric and water impermeable plastic film, a high waterproof melt blown nonwoven fabric, or an SMS (spun bonded/melt blown/spun bonded) nonwoven fabric having high strength spun bond nonwoven fabric sandwiching a melt blown nonwoven fabric. In an embodiment according to the present invention, as the non-skin contact side sheet 5, a 20 to 35 g/m² or so moisture permeable PE film is used. In some embodiments, the non-skin contact side sheet 5 may also be comprised of a material having moisture impermeability.

The absorptive body 6 has liquid retention ability and is comprised of an absorptive material. The absorptive material, for example, includes a fluff-like pulp or air laid nonwoven fabric and a granular high absorption polymer (or super absorption polymer, SAP). Here, the flap-shaped pulp is, for example, comprised of chemical pulp, cellulose fiber, rayon, acetate, or other artificial cellulose fiber, the air laid nonwoven fabric is, for example, comprised of pulp and synthetic fiber melt bonded or fixed by a binder to form a nonwoven fabric, and the SAP is, for example, comprised of a starch-based, acrylic acid-based, or amino acid-based granular or fibrous polymer. Note that, in an embodiment according to the present invention, the absorptive body 6 is formed by an absorptive material comprised of pulp and SAP wrapped by thin paper or a liquid permeable spun bonded nonwoven fabric, SMS nonwoven fabric, etc.

Here, if the mass ratio of the mass of the SAP to the mass of the absorptive material, that is, the total mass of the pulp and the SAP, is called the "SAP mass ratio", in an embodiment according to the present invention, the SAP mass ratio is set to 30 to 70 mass percent and, preferably, 35 to 55 mass percent. If the SAP mass ratio is smaller than 30 mass percent, it becomes difficult to secure an absorptive material density sufficient for imparting liquid diffusibility. If the SAP mass ratio is larger than 70 mass percent, the pulp or another fiber ingredient becomes relatively insufficient, a gel blocking phenomenon of the high absorption polymer easily occurs, and therefore the liquid diffusibility is likely to fall or, when worn, the absorptive body is likely to lose its shape due to the external force acting on the absorptive body. Note that the SAP mass ratio of a sanitary napkin is generally 0 to 10 mass percent, at most 20 mass percent, so the incontinence liner according to the present invention differs in constitution from a sanitary napkin in this respect.

The cushion sheet 7 is, for example, comprised of a nonwoven fabric made of a polyolefin-based thermoplastic hydrophobic fiber treated to be made hydrophilic. As the nonwoven fabric, an air-through nonwoven fabric, spun bonded nonwoven fabric, SMS nonwoven fabric, etc. may be used. In an embodiment according to the present invention, as the cushion sheet 7, a 20 to 40 g/m² air-through nonwoven fabric comprised of PE/PP fiber is used.

The side sheets 8 are, for example, comprised of a nonwoven fabric of a polyolefin-based thermoplastic hydrophobic fiber. As the nonwoven fabric, an air-through nonwoven fabric, spun bonded nonwoven fabric, SMS nonwoven fabric, etc. may be used. In an embodiment according to the present invention, as the side sheets 8, a 15 to 35 g/m² air-through nonwoven fabric comprised of PE/PP fibers is used.

The adhesives 9 and 10 are, for example, comprised of a styrene-isoprene-styrene block copolymer (SIS), styrene-butadiene-styrene block copolymer (SBS), styrene-ethylene-butylene-ethylene copolymer (SEBS), or another hot melt adhesive.

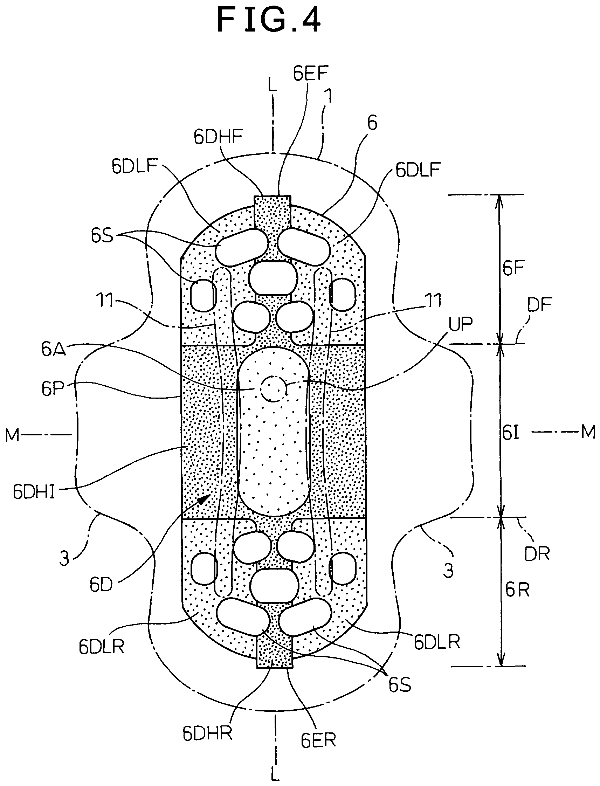

The absorptive body 6, as shown in FIG. 4, includes regions defined with respect to each other by defining (dividing) lines DF and DR extending in the width direction, that is, a front region 6F, a rear region 6R, and an intermediate region 6I between the front region 6F and the rear region 6R. In this case, the front region 6F is defined between a front edge 6EF of the absorptive body 6 and front defining line DF, the intermediate region 6I is defined between the front defining line DF and the rear defining line DR, and the rear region 6R is defined between the rear defining line DR and a rear edge 6ER of the absorptive body 6.

The center part of the intermediate region 6I in the longitudinal direction and width direction is provided with an oval-shaped absorbing part 6A extending along the longitudinal direction, while the intermediate region 6I around the absorbing part 6A is provided with a high diffusing part 6DHI. It is noted that the absorbing part may have an alternative, non-oval, shape.

The high diffusing part 6DHI may extend to the opposing side edges of the absorptive body 6 in the intermediate region 6I. In an embodiment, the absorbing part 6A and the high diffusing part 6DHI together cover the entirety of the intermediate region 6I.

The center parts of the front region 6F and the rear region 6R in the width direction are respectively provided with high diffusing parts 6DHF and 6DHR extending from the absorbing part 6A in the longitudinal direction. Further, the two sides of the high diffusing parts 6DHF and 6DHR, that is, peripheral parts of the front region 6F and the rear region 6R in the width direction, are respectively provided with low diffusing parts 6DLF and 6DLR. Note that, the high diffusing parts 6DHI, 6DHF, and 6DHR and the low diffusing parts 6DLF and 6DLR extend toward and preferably reach the entire peripheral edge 6P of the absorptive body 6.

In a preferred embodiment of the present invention, the high diffusing parts 6DHI 6DHF and 6DHR together surround the absorbing part 6A, with the high diffusing part 6DHI covering the entirety of the intermediate region 6I around the absorbing part 6A and extending to both edges of the absorptive body 6 in the intermediate region 6I, while the high diffusing part 6DHF projects from the high diffusing part 6DHI in a longitudinal direction along the centre of the front region 6F to the front edge 6EF of the absorptive body 6, such that it has a generally rectangular shape wherein its length in the longitudinal direction is greater than its width in the width direction, and the high diffusing part 6DHR projects from the high diffusing part 6DHI in a longitudinal direction along the centre of the rear region 6R to the rear edge 6ER of the absorptive body 6, such that it has a generally rectangular shape wherein its length in the longitudinal direction is greater than its width in the width direction. In this preferred arrangement, both the front and rear high diffusing parts 6DHF and 6DHR may be flanked on both sides by the low diffusing parts 6DLF and 6DLR respectively. However the invention is not to be limited to any particular shape of any of the diffusing parts, for example the high diffusing parts 6DHF and 6DHR need not be generally rectangular and may have another regular or irregular shape.

Furthermore, the front region 6F and the rear region 6R are provided with scattered diffusion-suppressing parts 6S. That is, in the high diffusing parts 6DHF and 6DHR and in the low diffusing parts 6DLF and 6DLR, the diffusion-suppressing parts 6S are provided discontinuously. Note that, in an embodiment according to the present invention, the diffusion-suppressing parts 6S are not provided at the intermediate region 6I. However, the diffusion-suppressing parts 6S may also be provided at the intermediate region 6I in other embodiments. Alternatively, the diffusion-suppressing parts 6S may also be provided only at the front region 6F or only at the rear region 6R.

Within the front region 6F, the diffusion-suppressing parts 6S when present are preferably present in both the high and low diffusion regions 6DHF and 6DLF, but may be present in only one of said regions. In a preferred embodiment, at least some of the diffusion-suppressing parts 6S overlap both the high and low diffusion regions 6DHF and 6DLF. The diffusion-suppressing parts 6S when present in the front region 6F preferably cover up to about 70% of the area of said front region 6F, for example up to 60%, or up to 50%, of the area of said front region 6F. The diffusion-suppressing parts 6S when present in the front region 6F preferably cover at least 10% of the area of said front region 6F, for example at least 20%, or at least 30%, of the area of said front region 6F.

In a preferred embodiment of the present invention, within the front region 6F, preferably, the high diffusion region 6DHF is discontinuous and divided into at least two parts by at least one of the diffusion-suppressing parts 6S, which avoids the possibility of quick diffusion of the liquid to be absorbed through any continuous paths in the high diffusion region 6DHF. In other words, any possible quick leakage paths through the high diffusion region 6DHF is blocked by at least one of the diffusion-suppressing parts 6S. For example, one of the diffusion-suppressing parts 6S (hereinafter referring as the blocking diffusion-suppressing part), which is at a center part of the front region 6F, can be seen from Fig.4. The blocking diffusion-suppressing part divides the high diffusion region 6DHF into two separate parts. As shown in Fig.4, even if the liquid quickly diffuses through the area between two diffusion-suppressing parts 6S which are close to the line DF, the diffusion rate of the liquid finally is slow down by the blocking diffusion-suppressing part 6S. Note that, the high diffusion region 6DHF divided by at least one of the diffusion-suppressing parts 6S is not an essential feature to solve the technical problem which the invention aims to solve, and the feature only makes a better effect on preventing the liquid leakage than that of a continuous high diffusion region 6DHF.

Within the rear region 6R, the diffusion-suppressing parts 6S when present are preferably present in both the high and low diffusion regions 6DHR and 6DLR, but may be present in only one of said regions. In a preferred embodiment, at least some of the diffusion-suppressing parts 6S overlap both the high and low diffusion regions 6DHR and 6DLR. The diffusion-suppressing parts 6S when present in the rear region 6R preferably cover up to about 70% of the area of said rear region 6R, for example up to 60%, or up to 50%, of the area of said rear region 6R. The diffusion-suppressing parts 6S when present in the rear region 6R preferably cover at least 10% of the area of said rear region 6R, for example at least 20%, or at least 30%, of the area of said rear region 6R.

In a preferred embodiment of the present invention, within the rear region 6R, preferably, the high diffusion region 6DHR is discontinuous and divided into at least two parts by at least one of the diffusion-suppressing parts 6S, which avoids the possibility of quick diffusion of the liquid to be absorbed through any continuous paths in the high diffusion region 6DHR. In other words, any possible quick leakage paths through the high diffusion region 6DHR is blocked by at least one of the diffusion-suppressing parts 6S. For example, one of the blocking diffusion-suppressing parts 6S, which is at a center part of the rear region 6R, can be seen form Fig.4. The blocking diffusion-suppressing part 6s divides the high diffusion region 6DHR into two separate parts. As shown in Fig.4, even if the liquid quickly diffuses through the area between two diffusion-suppressing parts 6S which are close to the line DR, the diffusion rate of the liquid finally is slow down by the blocking diffusion-suppressing part 6S. Note that, the high diffusion region 6DHR divided by at least one of the diffusion-suppressing parts 6S is not an essential feature to solve the technical problem which the invention aims to solve, and the feature only makes a better effect on preventing the liquid leakage than that of the continuous high diffusion region 6DHR.

In either of the front and rear regions, the diffusion-suppressing parts 6S may cover for example 10-70%, 10-60%, 10-50%, 20-70%, 20-60%, 20-50%, 30-70%, 30-60%, or 30-50%, of the area of said region.

The diffusion-suppressing parts can have several different sizes or have a uniform sizes. For example, in FIG.4, the sizes of the diffusion-suppressing parts 6S increase as the parts 6S are away from the intermediate region 6I.

Therefore, if considering that the diffusing part 6D is comprised of the high diffusing parts 6DHI, 6DHF, and 6DHR and low diffusing parts 6DLF and 6DLR, the absorptive body 6 can be said to include the absorbing part 6A, the diffusing part 6D extending around the absorbing part 6A up to the peripheral edge of the absorptive body 6, and the diffusion-suppressing parts 6S scattered in the diffusing part 6D. Further, the absorbing part 6A is provided inside the intermediate region 6I, the diffusing parts 6D are provided in the intermediate region 6I around the absorbing part 6A and at the front region 6F and the rear region 6R, and the diffusion-suppressing parts 6S are not provided at the intermediate region 6I, but are provided scattered in the diffusing part 6D at least at one of the front region 6F and the rear region 6R.

Note that, in an embodiment according to the present invention, the absorptive body 6 is formed symmetrically about the longitudinal direction center line L-L and the width direction center line M-M. In some embodiments, the absorptive body 6 is formed symmetrically only about the longitudinal direction center line L-L.

The absorbing part 6A, the high diffusing parts 6DHI, 6DHF, and 6DHR, the low diffusing parts 6DLF and 6DLR, and the diffusion-suppressing parts 6S differ in average absorptive material density. That is, if the average absorptive material density of a part x of the absorptive body 6 is expressed as the D(x), then D(6D)>D(6A) is established. Further, D(6S)<D(6D) is established. In particular, in an embodiment according to the present invention, D(6S)<D(6A) is established. Furthermore, D (6DHI, 6DHF, 6DHR) >D (6DLF, 6DLR) is established.

That is, D (6S) <D (6A) <D (6DLF, 6DLR) <D ( 6DHI, 6DHF, 6DHR) is established.

In some embodiments, preferably D (6A) =0.1 to 0.18 g/cm³, D (6DHI, 6DHF, 6DHR) =0.2 to 0.3 g/cm³, D (6DLF, 6DLR)=0.15 to 0.25 g/cm³, and D (6S) =0.01 to 0.09 g/cm³ are established. In an exemplary actual product, D(6A)=0.15 g/cm³, D(6DHI, 6DHF, 6DHR)=0.26 g/cm³, D(6DLF, 6DLR) =0.18 g/cm³, and D (6S) =0.03 g/cm³.

When worn, the main body 2 is fixed by the adhesives 9 to the inner surface of clothing such as underwear of the wearer, while the wings 3 are fixed to the outer surface of the clothing by the adhesives 10. That is, the main body 2 and wings 3 sandwich the clothing between them whereby the liner 1 is fastened to the inner side of the clothing. Note that, the clothing to which the liner 1 is fastened generally is panty-shaped with an elastic torso part and elastic leg parts. By fitting over the torso and legs of the wearer, the clothing as a whole fits against the skin of the wearer. As a result, the liner 1 fastened to the inner side of the clothing also is worn in a state fit along the skin of the wearer extending in the front-back direction centered at the crotch part.

In this case, the liner 1 is fastened at the crotch part of the clothing at the substantial center in the front-back direction. As a result, the urinating position UP of the wearer (FIG. 4) becomes positioned at the absorbing part 6A of the absorptive body 6, in particular at a position near the front region 6F in the absorbing part 6A. Therefore, the liquid to be absorbed mainly comprised of urine is mainly absorbed at the absorbing part 6A. The absorbing part 6A has a low absorptive material density, so the liquid to be absorbed slowly diffuses inside the absorbing part 6A. When the liquid to be absorbed next reaches the diffusing part 6D (high diffusing parts and low diffusing parts), since the diffusing part 6D is high in absorptive material density, the liquid to be absorbed is drawn into the diffusing part 6D by the capillary phenomenon and quickly diffuses inside the diffusing part 6D.

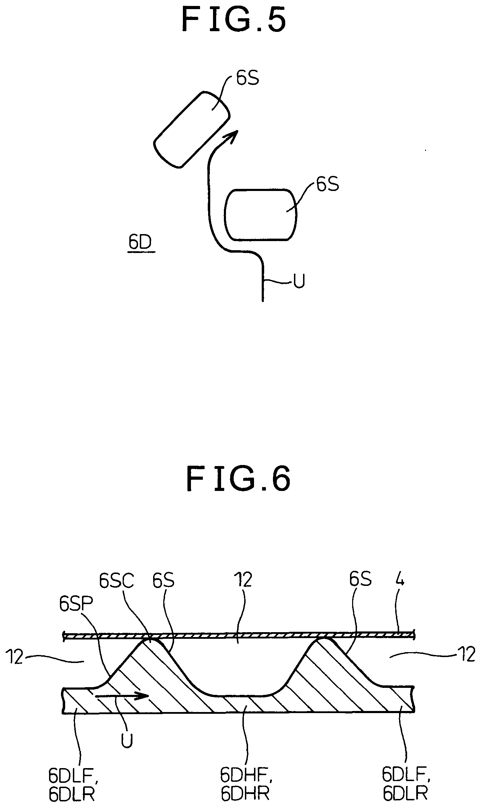

When the liquid to be absorbed next reaches the diffusion-suppressing parts 6S, since the diffusion-suppressing parts 6S are low in the absorptive material density, the liquid to be absorbed proceeds inside the diffusing part 6D while bypassing the diffusion-suppressing parts 6S, as shown in FIG. 5 by the arrow U. Alternatively, it slowly diffuses inside the diffusion-suppressing parts 6S. As a result, the diffusion speed of the liquid to be absorbed falls. Therefore, the liquid to be absorbed can be kept from leaking from the peripheral edge of the absorptive body 6.

Further, inside of the diffusing part 6D, the liquid to be absorbed flows from the high diffusing parts 6DHI, 6DHF, and 6DHR to the low diffusing parts 6DLF and 6DLR. Due to this as well, the diffusion speed of the liquid to be absorbed is kept down. This is because the low diffusing parts 6DLF and 6DLR are lower in average absorptive material density.

Here, the average absorptive material density D(x) (g/m³) of a part x of the absorptive body 6 is obtained by dividing the average basis weight (g/m²) of the absorptive material at that part x by the average thickness (mm) of that part x. In an embodiment according to the present invention, the absorptive material is comprised of pulp and SAP, so the basis weight of the absorptive material becomes the total of the basis weights of the pulp and SAP. The average thickness of the part x is obtained based on the measurements of the thickness of the part x other than the compression grooves 11.

The average absorptive material density of the absorptive body 6 is for example set as follows. That is, if expressing the average pulp basis weight and average SAP basis weight of a part x of the absorptive body 6 by BP(x) and BS(x), the absorptive body 6 is formed so that BP (6A) >BP (6D) >BP (6S) is established and so that BS (6A) >BS (6D) >BS (6S) is established. Therefore, if expressing the average absorptive material basis weight of a part x by B(x), the absorptive body 6 is formed so that B (6A) >B (6D) >B (6S) is established. Here, the average pulp basis weights and average SAP basis weights of the high diffusing parts 6DHI, 6DHF, and 6DHR are set to be substantially equal. The average absorptive material basis weights thereof are also set to be substantially equal.

Further, if expressing the average thickness of the part x by T(x), the absorptive body 6 is formed so that T (6A) >T (6DLF, 6DLR) >T (6DHI, 6DHF, 6DHR) is established. Here, the thicknesses of the high diffusing parts DHI, 6DHF, and 6DHR and the thicknesses of the low diffusing parts 6DLF and 6DLR are made substantially equal.

Note that, preferably T(6A)=4.0 to 6.0 mm, T(6DHI,6DHF,6DHR)=1 to 1.5 mm, T(6DLF, 6DLR)=1.2 to 2.2 mm, and T(6S)=2.5 to 3.5 mm are established. In an exemplary actual product, T(6A)=5.0 mm, T(6DHI, 6DHF, 6DHR)=1.3 mm, T(6DLF, 6DLR)=1.9 mm, and T(6S)=2.8 mm are established. Here, the thicknesses were measured using a thickness measuring device (Ozaki Manufacturing Co., Ltd., Dial Thickness Gauge Model H-0.4N, initial pressure of measurement force in specifications of "0.4N or less"). In this case, circular measurement pieces of 10mm in diameter were used.

An exemplary manufacturing process will be now described. The absorptive body 6 is, for example, formed by stacking pulp and SAP over a mesh pattern having air permeability. This mesh pattern is formed with a recessed part at a position corresponding to the absorbing part 6A and is formed with projecting parts at positions corresponding to the diffusion-suppressing parts 6S. As a result, the absorbing part 6A becomes larger in absorptive material basis weight, while the diffusion-suppressing parts 6S become smaller in absorptive material basis weight B. Next, press rollers having projections corresponding to the absorbing part 6A, low diffusing parts 6DLF and 6DLR, high diffusing parts 6DHI, 6DHF, and 6DHR, and diffusion-suppressing parts 6S are used to press the absorptive body 6. For example, the projections corresponding to the high diffusing parts 6DHI, 6DHF, and 6DHR are made high in height, while the projections corresponding to the diffusion-suppressing parts 6S are made low in height. As a result, the high diffusing parts 6DHI, 6DHF, and 6DHR become smaller in thickness and the diffusion-suppressing parts 6S become greater in height (thickness). Note that, due to the above-mentioned pressing, sometimes slight relief shapes are formed at one or both of the front and back surfaces of the absorptive material 6 and therefore the parts x of the absorptive material 6 are not uniform in thickness. For this reason, in the above explanation, the average thickness is used.

In this case, as shown in FIG. 2, the absorbing part 6A projects toward the skin contact side sheet 4, that is, toward the wearer, more than the other parts of the absorptive body 6. As a result, the absorbing part 6A reliably abuts against the urinating position UP of the wearer (FIG. 4), and the liquid to be absorbed is reliably absorbed from the absorbing part 6A.

Further, as shown in FIG. 6, the diffusion-suppressing parts 6S project toward the skin contact side sheet 4, that is, toward the wearer, more than the adjoining high diffusing parts 6DHF and 6DHR and low diffusing parts 6DLF and 6DLR. As a result, even if the liquid to be absorbed diffuses inside the diffusion-suppressing parts 6S, the liquid to be absorbed, as shown by the arrow U, mainly diffuses through the bottoms of the diffusion-suppressing parts 6S and does not diffuse much through the tops of the diffusion-suppressing parts 6S. As a result, even if the liner 1 is pressed against the wearer, the once absorbed liquid to be absorbed can be kept from depositing on the skin of the wearer and therefore the skin of the wearer can be kept in the dry state. In particular, a protruding part of the body of the wearer tends to strike the front region 6F causing pressure to act thereon, so the front region 6F is preferably provided with diffusion-suppressing parts 6S. Further, when the wearer sits down or squats down, pressure tends to act on the rear region 6R, so the rear region 6R is also preferably provided with diffusion-suppressing parts 6S.

Further, as shown in FIG. 6, the diffusion-suppressing parts 6S are tapered upwardly in the thickness direction. In this case, for example, the center parts 6SC of the diffusion-suppressing parts 6S are larger in thickness than the surrounding parts 6SP. Therefore, the center parts 6SC have an average absorptive material density lower than the average absorptive material density of the surrounding parts 6SP. As a result, even if the liquid to be absorbed diffuses in the diffusion-suppressing parts 6S, the liquid to be absorbed mainly diffuses inside of the surrounding parts 6SP and does not diffuse much at all inside of the center parts 6SC including the tops.

Furthermore, between adjacent diffusion-suppressing parts 6S and between the skin contact side sheet 4 and diffusing part 6D (high diffusing part and low diffusing part), clearances 12 are formed. As a result, a return of the absorbed liquid from the absorptive body 6 to the skin contact side sheet 4 can be further suppressed and the skin of the wearer can be kept in a dry state over a long period of time.

Referring again to FIG. 4, the above-mentioned wings 3 are positioned adjoining the intermediate region 6I of the absorptive body 6. In this case, the intermediate region 6I is provided with the high diffusing part 6DHI which is relatively high density and therefore relatively hard. This hard portion can resist body pressure or bending force resulting from movement by the wearer while worn. As a result, when worn, the liner 1 is kept from wrinkling or twisting near the wings 3, therefore the liner 1 can continue to be reliably fastened to the clothing over a long period of time. Note that, in general, the frequency of changing the incontinence liner 1 is lower than that of a sanitary napkin, therefore the wearing time of the liner 1 is longer, so reliable fastening of the liner 1 over a long period is required.

Further, three relatively hard high diffusing parts 6DHI, 6DHF, and 6DHR are arranged along the longitudinal direction, that is, the front-back direction of the wearer. As a result, wrinkles or twisting can be kept from occurring at the adhesives 9 (FIG. 3) extending along the longitudinal direction, therefore the wearer can easily fasten the liner 1 to the clothing. Further, the liner 1 as a whole can be kept from wrinkling or twisting, so diffusion of the liquid to be absorbed at the absorptive body 6 can be realized as targeted.

Furthermore, as shown in FIG. 4, the above-mentioned compression grooves 11 extend from the intermediate region 6I at the two sides of the absorbing part 6A up to the front region 6F and the rear region 6R. As a result, the absorptive body 6 can be bent along the compression grooves 11, and the liner 1 can deform tracking the body of the wearer. Further, the compression grooves 11 are relatively high in absorptive material density, so the liquid to be absorbed diffuses inside of the compression grooves 11 as well to reach the front region 6F and the rear region 6R where it is further diffused.

On the other hand, the front region 6F and the rear region 6R are provided with the diffusion-suppressing parts 6S which are relatively low in density and therefore relatively soft. As a result, the liner 1 can deform tracking the body of the wearer. In this case, since the diffusion-suppressing parts 6S are provided scattered about, that is, are provided discontinuously, the front region 6F and the rear region 6R do not become excessively soft and undesirable wrinkling and twisting of the front region 6F and the rear region 6R can be kept from occurring.

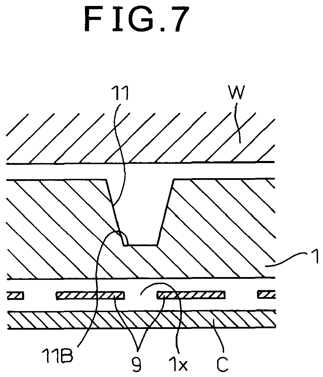

Here, referring to FIG. 7 and FIG. 3, the adhesives 9 are positioned at the two sides of the areas around the front edges 11F of the compression grooves 11 in the width direction and extend beyond the front edges 11F toward the front in the longitudinal direction. Similarly, the adhesives 9 are positioned at the two sides of the areas around the rear edges 11R of the compression grooves 11 in the width direction and extend beyond the rear edges 11R toward the rear in the longitudinal direction.

By this configuration, the areas around the front edges 11F and the areas around the rear edges 11R of the compression grooves 11 are surrounded by the two adhesives 9. As a result, even if the liner 1 is bent along the compression grooves 11, the liner 1 can continue to be reliably fastened to the clothing C and therefore the liner 1 can be kept from wrinkling or twisting.

On the other hand, the adhesives 9 do not overlap the areas around the front edges 11F and rear edges 11R of the compression grooves 11, in particular the bottoms 11B. That is, the outer surfaces of the liner 1 or non-skin contact side sheet 5 facing the compression grooves 11, in particular their bottoms 11B, are provided with adhesive-free regions 1x. As a result, a deformation of the liner 1 along with compression grooves 11 is not inhibited by the adhesives 9. Therefore, when the liner 1 deforms when worn, the liner 1 can easily track the body of the wearer.

In addition, referring to FIG.4, the parts of the compression grooves 11 at the front region 6F and/or at the rear region 6R are surrounded by the diffusion-suppressing parts 6S, and the diffusion-suppressing parts 6S substantially do not overlap the compression grooves 11. As a result, the diffusion rate of the liquid to be absorbed in the pair of compression grooves 11 of the absorptive body 6 will not be slowed down by any diffusion-suppressing part 6S, and it also facilitates the manufacture process.

Note that, the above-mentioned PLT 1 discloses scattered space layers. The space layers may appear to correspond to the diffusion-suppressing parts of the present invention. However, PLT 1 does not consider the absorptive material density at all. It is not known if the absorptive material density of the space layers is lower than the absorptive material density of the layers surrounding them. Further, PLT 1 does not disclose the technical problem of suppressing the diffusion speed of the liquid to be absorbed at all and naturally does not disclose a technical solution to this.

By "skin contact side sheet (skin contacting sheet)" is meant the sheet positioned on the skin contact side of the incontinence liner, and by "non-skin contact side sheet (non-skin contacting sheet)" is meant the sheet positioned on the non-skin contact side of the incontinence liner.

By "projects toward the skin contact side sheet" is meant that, in the case of the absorbing part, in cross-section the absorbing part is thicker than the other parts of the absorptive body such that its upper side is closer to the wearer's body than the other parts when in use; and in the case of the diffusing suppressing part, in cross-section the diffusion-suppressing parts are thicker than the diffusing part such that their upper sides are closer to the wearer's body than the diffusing part when in use.

This application claims the benefit of Japanese Application No. 2010-194643 the entire disclosure of which is incorporated by reference herein.

### Reference Signs List

- 1: incontinence liner
- 2: main body
- 3: wings
- 4: skin contact side sheet
- 5: non-skin contact side sheet
- 6: absorptive body
- 6A: absorbing part
- 6D: diffusing part
- 6S: diffusion-suppressing part
- 7: cushion sheet
- 8: side sheet
- 9, 10: adhesive
- 11: compression grooves
- 12: clearance

## Claims

1. An incontinence liner (1) provided with a liquid permeable skin contacting sheet (4), a liquid impermeable non-skin contacting sheet (5), and an absorptive body (6) arranged between the skin contacting sheet (4) and the non-skin contacting sheet (5) including an absorptive material comprising 30 to 70 mass% of a high absorption polymer, wherein
said absorptive body (6) includes:
a front region (6F), a rear region (6R), and an intermediate region (6I) between the front region (6F) and the rear region (6R);
an absorbing part (6A) provided at said intermediate region (6I) and adapted to be positioned at a urinating position (UP) of a wearer (W) when worn;
high diffusing parts (6DHI, 6DHF, 6DHR) provided at said intermediate region (6I) around said absorbing part (6A) and at center parts in the width direction of said front region (6F) and rear region (6R);
low diffusing parts (6DLF, 6DLR) provided at peripheral parts in the width direction of said front region (6F) and rear region (6R); and
diffusion-suppressing parts (6S) not provided at said intermediate region and provided scattered in the diffusing parts (6D) in at least one of said front region (6F) and rear region (6R), said high diffusing parts (6DHI, 6DHF, 6DHR) and low diffusing parts (6DLF, 6DLR) extending up to the peripheral edge (6P) of the absorptive body (6A);
the average absorptive material density of the high diffusing parts (6DHI, 6DHF, 6DHR) and low diffusing parts (6DLF, 6DLR) being higher than the average absorptive material density of said absorbing part (6A);
the average absorptive material density of the diffusion-suppressing parts (6S) being lower than the average absorptive material density of the high diffusing parts (6DHI, 6DHF, 6DHR) and low diffusing parts (6DLF, 6DLR);
and the average absorptive material density of said high diffusing parts (6DHI, 6DHF, 6DHR) being higher than an average absorptive material density of said low diffusing parts (6DLF, 6DLR); and
wherein the absorbing part (6A) projects toward the skin contacting sheet (4) more than the other parts of the absorptive body (6A); and the diffusion-suppressing parts (6S) project out toward the skin contacting sheet (4) more than the diffusing parts (6D);
said diffusion-suppressing parts (6S) are tapered;
said liner (1) is provided with a pair of wings (3) for fastening said liner (1) to clothing (C), said wings (3) provided adjoining said intermediate region (6I); and
said liner (1) is provided with a pair of compression grooves (11) extending from the two sides of said absorbing part (6A) up to said front region (6F) and rear region (6R).

2. An incontinence liner (1) as in Claim 1, wherein the average absorptive material density of said diffusion-suppressing parts (6S) is lower than the average absorptive material density of said absorbing part (6A).

3. An incontinence liner as in Claim 1 or Claim 2, wherein said high diffusing part in the intermediate region (6DHI) extends to the opposing side edges of the absorptive body 6 the absorbing part 6A and the high diffusing part 6DHI together cover the entirety of the intermediate region 6I.

4. An incontinence liner as in any one of Claim 1 to Claim 3, wherein the thickness of the absorbing part (6A) of the absorptive body 6 is greater than the thicknesses of the low-diffusing parts in the front and rear regions (6DLF, 6DLR); the thicknesses of the low-diffusing parts in the front and rear regions (6DLF, 6DLR) are greater than the thicknesses of the high-diffusing parts in the front, rear and intermediate regions (6DHF, 6DHR, 6DHI); the thicknesses of the low-diffusing parts in the front and rear regions (6DLF, 6DLR) are substantially equal; and the thicknesses of the high-diffusing parts in the front, rear and intermediate regions (6DHF, 6DHR, 6DHI)are substantially equal.

5. An incontinence liner (1) as in any one of Claim 1 to Claim 4, wherein the high-diffusing parts in the front, rear and intermediate regions (6DHF, 6DHR, 6DHI) are provided arranged along the longitudinal direction, that is, in the front-back direction of the wearer.

6. An incontinence liner (1) as in any one of Claim 1 to Claim 5, wherein said diffusion-suppressing parts (6S) are tapered such that centre parts (6SC) of said diffusion-suppressing parts (6S) are larger in thickness than the surrounding parts (6SP).

7. An incontinence liner (1) as in Claim 6, wherein clearances (12) are formed between said tapered diffusion-suppressing parts (6S) and the skin contacting sheet 4.

8. An incontinence liner (1) as in any one of Claim 1 to Claim 7, wherein belt-shaped adhesives (9) separated from each other in the width direction and extending in parallel in the longitudinal direction are applied at the outer surface of the non-skin contacting sheet (5) corresponding to the back surface of the main body (2).

9. An incontinence liner (1) as in Claim 8, wherein the belt-shaped adhesives (9) are positioned at the two sides of the areas around the front edges (11F) of the compression grooves (11) in the width direction and extend over the front edges (11F) to the front in the longitudinal direction; and at the two sides of the areas around the rear edges (11R) of the compression grooves (11) in the width direction and extend over the rear edges (11R) to the rear in the longitudinal direction; such that the areas around the front edges (11F) and the areas around the rear edges (11R) of the compression grooves (11) are surrounded by the adhesives (9).

10. An incontinence liner (1) as in Claim 9, wherein the adhesives (9) do not overlap the areas around the front edges (11F) and rear edges (11R) of the compression grooves (11), in particular the bottoms (11B), such that the outer surfaces of the liner (1) or non-skin contacting sheet (5) facing the compression grooves (11), in particular their bottoms (11B), are provided with adhesive-free regions (1x).

## Patentansprüche

1. Inkontinenzeinlage (1), die mit Folgendem bereitgestellt ist: einer flüssigkeitsdurchlässigen, Haut berührenden Lage (4), einer flüssigkeitsundurchlässigen, nicht die Haut berührenden Lage (5) und einem absorbierenden Körper (6), der zwischen der Haut berührenden Lage (4) und der nicht die Haut berührenden Lage (5) angeordnet ist, ein absorbierendes Material einschließt, das 30 bis 70 Masse-% eines hochabsorbierenden Polymers umfasst, wobei
der absorbierende Körper (6) Folgendes einschließt:
einen vorderen Bereich (6F), einen hinteren Bereich (6R) und einen Zwischenbereich (6I) zwischen dem vorderen Bereich (6F) und dem hinteren Bereich (6R);
einen absorbierenden Teil (6A), der an dem Zwischenbereich (6I) bereitgestellt ist und zur Positionierung an einer urinierenden Position (UP) eines Trägers (W) beim Tragen geeignet ist;
Teile mit hoher Diffusion (6DHI, 6DHF, 6DHR), die an dem Zwischenbereich (6I) um den absorbierenden Teil (6A) und an mittleren Teilen in der Breitenrichtung des vorderen Bereichs (6F) und hinteren Bereichs (6R) bereitgestellt sind;
Teile mit niedriger Diffusion (6DLF, 6DLR), die an peripheren Teilen in der Breitenrichtung des vorderen Bereichs (6F) und hinteren Bereichs (6R) bereitgestellt sind; und
Diffusion unterdrückende Teile (6S), die nicht an dem Zwischenbereich bereitgestellt sind und verstreut in den Teilen mit Diffusion (6D) in mindestens einem von dem vorderen Bereich (6F) und hinteren Bereich (6R) bereitgestellt sind, wobei die Teile mit hoher Diffusion (6DHI, 6DHF, 6DHR) und Teile mit niedriger Diffusion (6DLF, 6DLR) sich bis zum peripheren Rand (6P) des absorbierenden Körpers (6A) erstrecken;
die durchschnittliche Dichte des absorbierenden Materials der Teile mit hoher Diffusion (6DHI, 6DHF, 6DHR) und Teile mit niedriger Diffusion (6DLF, 6DLR) höher ist als die durchschnittliche Dichte des absorbierenden Materials des absorbierenden Teils (6A);
die durchschnittliche Dichte des absorbierenden Materials der Diffusion unterdrückenden Teile (6S) niedriger ist als die durchschnittliche Dichte des absorbierenden Materials der Teile mit hoher Diffusion (6DHI, 6DHF, 6DHR) und Teile mit niedriger Diffusion (6DLF, 6DLR);
und die durchschnittliche Dichte des absorbierenden Materials der Teile mit hoher Diffusion (6DHI, 6DHF, 6DHR) höher ist als eine durchschnittliche Dichte des absorbierenden Materials der Teile mit niedriger Diffusion (6DLF, 6DLR); und
wobei der absorbierende Teil (6A) in Richtung der Haut berührenden Lage (4) mehr herausragt als die anderen Teile des absorbierenden Körpers (6A); und die Diffusion unterdrückenden Teile (6S) in Richtung der Haut berührenden Lage (4) mehr herausragen als die Teile mit Diffusion (6D);
die Diffusion unterdrückenden Teile (6S) konisch sind;
die Einlage (1) mit einem Paar an Flügeln (3) zur Befestigung der Einlage (1) an der Bekleidung (C) bereitgestellt ist, die Flügel (3) angrenzend an den Zwischenbereich (6I) bereitgestellt sind; und
die Einlage (1) mit einem Paar an Kompressionsrillen (11) bereitgestellt ist, die sich von den zwei Seiten des absorbierenden Teils (6A) bis zu dem vorderen Bereich (6F) und hinteren Bereich (6R) erstrecken.

2. Inkontinenzeinlage (1) nach Anspruch 1, wobei die durchschnittliche Dichte des absorbierenden Materials der Diffusion unterdrückenden Teile (6S) niedriger ist als die durchschnittliche Dichte des absorbierenden Materials des absorbierenden Teils (6A).

3. Inkontinenzeinlage nach Anspruch 1 oder Anspruch 2, wobei der Teil mit hoher Diffusion in dem Zwischenbereich (6DHI) sich zu den gegenüberliegenden Seitenrändern des absorbierenden Körpers 6 erstreckt, der absorbierende Teil 6A und der Teil mit hoher Diffusion 6DHI zusammen die Gesamtheit des Zwischenbereichs 6I abdecken.

4. Inkontinenzeinlage nach einem von Anspruch 1 bis Anspruch 3, wobei die Dicke des absorbierenden Teils (6A) des absorbierenden Körpers 6 größer ist als die Dicken der Teile mit niedriger Diffusion in dem vorderen und hinteren Bereich (6DLF, 6DLR); die Dicken der Teile mit niedriger Diffusion in dem vorderen und hinteren Bereich (6DLF, 6DLR) größer sind als die Dicken der Teile mit hoher Diffusion in dem vorderen, hinteren und Zwischenbereich (6DHF, 6DHR, 6DHI); die Dicken der Teile mit niedriger Diffusion in dem vorderen und hinteren Bereich (6DLF, 6DLR) im Wesentlichen gleich sind; und die Dicken der Teile mit hoher Diffusion in dem vorderen, hinteren und Zwischenbereich (6DHF, 6DHR, 6DHI) im Wesentlichen gleich sind.

5. Inkontinenzeinlage (1) nach einem von Anspruch 1 bis Anspruch 4, wobei die Teile mit hoher Diffusion in dem vorderen, hinteren und Zwischenbereich (6DHF, 6DHR, 6DHI) entlang der Längsrichtung angeordnet bereitgestellt sind, das heißt in der Vorderseite-Rückseite-Richtung des Trägers.

6. Inkontinenzeinlage (1) nach einem von Anspruch 1 bis Anspruch 5, wobei die Diffusion unterdrückenden Teile (6S) konisch sind, sodass mittlere Teile (6SC) der Diffusion unterdrückenden Teile (6S) größer sind in der Dicke als die umgebenden Teile (6SP).

7. Inkontinenzeinlage (1) nach Anspruch 6, wobei zwischen den konischen, Diffusion unterdrückenden Teilen (6S) und der Haut berührenden Lage 4 Freiräume (12) ausgebildet sind.

8. Inkontinenzeinlage (1) nach einem von Anspruch 1 bis Anspruch 7, wobei bandförmige Haftmittel (9), die in der Breitenrichtung voneinander getrennt sind und sich parallel in der Längsrichtung erstrecken, auf der äußeren Oberfläche der nicht die Haut berührenden Lage (5), die der Rückseite des Hauptkörpers (2) entspricht, aufgetragen sind.

9. Inkontinenzeinlage (1) nach Anspruch 8, wobei die bandförmigen Haftmittel (9) an den zwei Seiten der Flächen um die vorderen Ränder (11F) der Kompressionsrillen (11) in der Breitenrichtung positioniert sind und sich über die vorderen Ränder (11F) bis zur Vorderseite in der Längsrichtung erstrecken; und an den zwei Seiten der Flächen um die hinteren Ränder (11R) der Kompressionsrillen (11) in der Breitenrichtung positioniert sind und sich über die hinteren Ränder (11R) bis zur Hinterseite in der Längsrichtung erstrecken; sodass die Flächen um die vorderen Ränder (11F) und die Flächen um die hinteren Ränder (11R) der Kompressionsrillen (11) von den Haftmitteln (9) umgeben sind.

10. Inkontinenzeinlage (1) nach Anspruch 9, wobei die Haftmittel (9) nicht mit den Flächen um die vorderen Ränder (11F) und hinteren Ränder (11R) der Kompressionsrillen (11), insbesondere den Unterseiten (11B), überlappen, sodass die äußeren Oberflächen der Einlage (1) oder nicht die Haut berührenden Lage (5), die den Kompressionsrillen (11), insbesondere ihren Unterseiten (11B), zugewandt sind, mit Haftmittel-freien Bereichen (1x) bereitgestellt sind.

## Revendications

1. Serviette de protection pour l'incontinence (1) comportant une feuille perméable aux liquides en contact avec la peau (4), une feuille imperméable aux liquides non en contact avec la peau (5), et un corps absorbant (6) agencé entre la feuille en contact avec la peau (4) et la feuille non en contact avec la peau (5) comprenant une matière absorbante comportant de 30 à 70 % en masse d'un polymère à haute absorption, dans laquelle
ledit corps absorbant (6) comprend :
une région avant (6F), une région arrière (6R), et une région intermédiaire (6I) entre la région avant (6F) et la région arrière (6R) ;
une partie absorbante (6A) mise en oeuvre au niveau de ladite région intermédiaire (6I) et adaptée pour être positionnée au niveau d'une position de miction (UP) d'un utilisateur (W) quand elle est portée ;
des parties de haute diffusion (6DHI, 6DHF, 6DHR) mises en oeuvre au niveau de ladite région intermédiaire (6I) autour de ladite partie absorbante (6A) et au niveau de parties centrales dans la direction allant dans le sens de la largeur de ladite région avant (6F) et de ladite région arrière (6R) ;
des parties de faible diffusion (6DLF, 6DLR) mises en oeuvre au niveau de parties périphériques dans la direction allant dans le sens de la largeur de ladite région avant (6F) et de ladite région arrière (6R) ; et
des parties de suppression de diffusion (6S) non mises en oeuvre au niveau de ladite région intermédiaire et mises en oeuvre de manière dispersée dans les parties de diffusion (6D) dans au moins l'une parmi ladite région avant (6F) et ladite région arrière (6R), lesdites parties de haute diffusion (6DHI, 6DHF, 6DHR) et lesdites parties de faible diffusion (6DLF, 6DLR) s'étendant jusqu'au bord périphérique (6P) du corps absorbant (6A) ;
la densité moyenne de la matière absorbante des parties de haute diffusion (6DHI, 6DHF, 6DHR) et des parties de faible diffusion (6DLF, 6DLR) étant supérieure par rapport à la densité moyenne de la matière absorbante de ladite partie absorbante (6A) ;
la densité moyenne de la matière absorbante des parties de suppression de diffusion (6S) étant inférieure par rapport à la densité moyenne de la matière absorbante des parties de haute diffusion (6DHI, 6DHF, 6DHR) et des parties de faible diffusion (6DLF, 6DLR) ;
et la densité moyenne de la matière absorbante desdites parties de haute diffusion (6DHI, 6DHF, 6DHR) étant supérieure par rapport à une densité moyenne de la matière absorbante desdites parties de faible diffusion (6DLF, 6DLR) ; et
dans laquelle la partie absorbante (6A) fait plus saillie vers la feuille en contact avec la peau (4) par rapport aux autres parties du corps absorbant (6A) ; et les parties de suppression de diffusion (6S) font plus saillie vers l'extérieur vers la feuille en contact avec la peau (4) par rapport aux parties de diffusion (6D) ;
lesdites parties de suppression de diffusion (6S) sont coniques ;
ladite serviette de protection (1) comporte une paire d'ailettes (3) servant à des fins de fixation de ladite serviette de protection (1) sur le vêtement (C), lesdites ailettes (3) étant mises en oeuvre de manière contiguë par rapport à ladite région intermédiaire (6I) ; et
ladite serviette de protection (1) comporte une paire de rainures de compression (11) s'étendant depuis les deux côtés de ladite partie absorbante (6A) jusqu'à ladite région avant (6F) et ladite région arrière (6R).

2. Serviette de protection pour l'incontinence (1) selon la revendication 1, dans laquelle la densité moyenne de la matière absorbante desdites parties de suppression de diffusion (6S) est inférieure par rapport à la densité moyenne de la matière absorbante de ladite partie absorbante (6A).

3. Serviette de protection pour l'incontinence selon la revendication 1 ou la revendication 2, dans laquelle ladite partie de haute diffusion dans la région intermédiaire (6DHI) s'étend jusqu'aux bords latéraux opposés du corps absorbant 6, la partie absorbante 6A et la partie de haute diffusion 6DHI recouvrent ensemble la totalité de la région intermédiaire 6I.

4. Serviette de protection pour l'incontinence selon l'une quelconque des revendications 1 à 3, dans laquelle l'épaisseur de la partie absorbante (6A) du corps absorbant 6 est supérieure par rapport aux épaisseurs des parties de faible diffusion dans les régions avant et arrière (6DLF, 6DLR) ; les épaisseurs des parties de faible diffusion dans les régions avant et arrière (6DLF, 6DLR) sont supérieures par rapport aux épaisseurs des parties de haute diffusion dans les régions avant, arrière et intermédiaire (6DHF, 6DHR, 6DHI) ; les épaisseurs des parties de faible diffusion dans les régions avant et arrière (6DLF, 6DLR) sont sensiblement égales ; et les épaisseurs des parties de haute diffusion dans les régions avant, arrière et intermédiaire (6DHF, 6DHR, 6DHI) sont sensiblement égales.

5. Serviette de protection pour l'incontinence (1) selon l'une quelconque des revendications 1 à 4, dans laquelle les parties de haute diffusion dans les régions avant, arrière et intermédiaire (6DHF, 6DHR, 6DHI) sont mises en oeuvre selon un agencement le long de la direction allant dans le sens longitudinal, à savoir, dans une direction allant dans le sens avant-arrière de l'utilisateur.

6. Serviette de protection pour l'incontinence (1) selon l'une quelconque des revendications 1 à 5, dans laquelle lesdites parties de suppression de diffusion (6S) sont coniques de telle sorte que les parties centrales (6SC) desdites parties de suppression de diffusion (6S) sont plus larges dans l'épaisseur par rapport aux parties environnantes (6SP).

7. Serviette de protection pour l'incontinence (1) selon la revendication 6, dans laquelle des dégagements (12) sont formés entre lesdites parties de suppression de diffusion coniques (6S) et la feuille en contact avec la peau 4.

8. Serviette de protection pour l'incontinence (1) selon l'une quelconque des revendications 1 à 7, dans laquelle des adhésifs en forme de ceinture (9) séparés les uns des autres dans la direction allant dans le sens de la largeur et s'étendant de manière parallèle dans la direction allant dans le sens longitudinal sont appliqués au niveau de la surface extérieure de la feuille non en contact avec la peau (5) correspondant à la surface arrière du corps principal (2).

9. Serviette de protection pour l'incontinence (1) selon la revendication 8, dans laquelle les adhésifs en forme de ceinture (9) sont positionnés au niveau des deux côtés des zones autour des bords avant (11F) des rainures de compression (11) dans la direction allant dans le sens de la largeur et s'étendent par-dessus les bords avant (11F) jusqu'à la partie avant dans la direction allant dans le sens longitudinal ; et au niveau des deux côtés des zones autour des bords arrière (11R) des rainures de compression (11) dans la direction allant dans le sens de la largeur et s'étendent par-dessus les bords arrière (11R) jusqu'à la partie arrière dans la direction allant dans le sens longitudinal ; de telle sorte que les zones autour des bords avant (11F) et les zones autour des bords arrière (11R) des rainures de compression (11) sont entourées par les adhésifs (9).

10. Serviette de protection pour l'incontinence (1) selon la revendication 9, dans laquelle les adhésifs (9) ne chevauchent pas les zones autours des bords avant (11F) et des bords arrière (11R) des rainures de compression (11), en particulier les parties inférieures (11B), de telle sorte que les surfaces extérieures de la serviette de protection (1) ou de la feuille non en contact avec la peau (5) faisant face aux rainures de compression (11), en particulier leurs parties inférieures (11B), comportent des régions exemptes d'adhésif (1x).
